# EUROPEAN PATENT SPECIFICATION

(11) **EP 3 186 362 B1**
(45) Date of publication and mention of the grant of the patent: **15.05.2019**
(21) Application number: 15797403.1
(22) Date of filing: 31.08.2015
(51) Int. Cl.: C12N 1/20, C12N 7/00, C12R 1/445

(54) **STAPHYLOCOCCUS AUREUS STRAINS FOR PRODUCTION OF MONOCLONAL PHAGE PREPARATIONS DEPRIVED OF CONTAMINATION WITH PLASMID DNA**
STAPHYLOCOCCUS AUREUS STÄMME ZUR HERSTELLUNG MONOKLONALER ANTIKÖRPER OHNE VERUNREINIGUNG MIT PLASMID DNA
SOUCHES DE STAPHYLOCOCCUS AUREUS POUR LA PRODUCTION D'ANTICORPS MONOCLONAUX SANS CONTAMINATION AVEC DE L'ADN PLASMIDIQUE

(30) Priority: 31.08.2014 PL 40933014
(43) Date of publication of application: 05.07.2017
(60) Divisional application: 18195142.7
(73) Proprietor: Instytut Biochemii I Biofizyki Polskiej Akademii Nauk, 02-106 Warszawa (PL); Instytut Immunologii I Terapii Doswiadczalnej Polskiej Akademii, PL-53-114 Wroclaw (PL)
(72) Inventor: LOBOCKA, Malgorzata, PL-02-798 Warszawa (PL); HEJNOWICZ, Monika, PL-02-695 Warszawa (PL); DABROWSKI, Kamil, PL-05-071 Sulejówek (PL); IZAK, Dariusz, PL-05-803 Pruszków (PL); GOZDEK, Agnieszka, PL-00-714 Warszawa (PL); GLOWACKA, Aleksandra, PL-02-106 Warszawa (PL); GAWOR, Jan, PL-04-357 Warszawa (PL); KOSAKOWSKI, Jaroslaw, PL-02-796 Warszawa (PL); GROMADKA, Robert, PL-01-960 Warszawa (PL); WEBER-DABROWSKA, Beata, PL-51-628 Wroclaw (PL); GÓRSKI, Andrzej, PL-02-784 Warszawa (PL)
(74) Representative: Witek, Rafal
(86) International application number: PCT/IB2015/056606
(87) International publication number: WO 2016/030871

(56) References cited:
- WO-A2-2014/048407
- Pierre Vaudaux ET AL: "Increased Expression of Clumping Factor and Fibronectin-Binding Proteins by hemB Mutants of Staphylococcus aureus Expressing Small Colony Variant Phenotypes", INFECTION AND IMMUNITY, 1 October 2002 (2002-10-01), pages 5428-5437, XP055021599, Retrieved from the Internet: URL:http://www.ncbi.nlm.nih.gov/pmc/articl es/PMC128368/pdf/1704.pdf [retrieved on 2012-03-12]

## Description

The object of the invention is *Staphylococcus aureus* strain for obtaining monoclonal preparations of lytic bacteriophages with high titer, devoid of contamination with undesired bacteriophages and plasmid DNA. The strain being the result of the invention guarantees the production of safe to use bacteriophage preparations, not being a source of dissemination of genes associated with bacterial virulence.

While facing a crisis of effectiveness of antibiotic therapy for bacterial infections, in recent years a return to studies on phage therapy as an alternative method for treating infections is observed (Górski et al., 2009; Golka et al., 2014). The therapeutic agents in phage therapy are bacteriophages - viruses that selectively infect bacteria and are harmless to eukaryotic organisms. Bacteriophages, as obligate bacterial parasites, can to obtained only by multiplication in bacterial cells. The release from infected cells of the bacteriophages multiplied therein is correlated with cell lysis and spilling out their contents to the environment. The thus obtained lysates contain bacteriophages suspended in a mixture of cell contents and the medium wherein the bacteria were cultured. The safety of phage therapy requires them to be free from factors harmful to organisms treated with the bacteriophages, as well as of factors capable of increasing pathogenicity of bacteria. The hitherto methods of producing therapeutic phage preparations, *inter alia* with phages against *Staphylococcus aureus,* do not fully ensure such safety due to the lack of suitable bacterial strains for phage multiplication.

Bacteriophages that are effective in fighting infections caused by bacterial pathogens typically do not multiply in cells of non-pathogenic bacteria. Moreover, preparations with high titer, lytically effective against the particular pathogen, can be most often obtained only by multiplication of the given phage in bacteria of the same species, representing strains having genetic features similar to the fought pathogen (D'Herelle, 1938; Balogh et al., 2010). They therefore contain contaminations that are derived from the cells of bacterial pathogens. These bacteria encode numerous virulence factors, such as e.g. bacterial toxins. Moreover, the genes encoding these factors are usually a part of mobile genetic elements present in the cells, capable of transferring by various methods from one bacterial cell to another.

The most difficult to eliminate from phage preparations are the contaminations with bacteriophages different than the ones multiplied. These bacteriophages belong to the so-called temperate bacteriophages, being a specific type of mobile genetic elements (Guttman et al., 2005). As opposed to obligate lytic bacteriophages recommended for therapeutic applications and multiplying only through the lytic development pathway, temperate bacteriophages can multiply through the lytic development pathway or remain in the cell in the form of a so-called prophage - a DNA molecule generally integrated into the bacterial chromosome. They can remain in this form for years, replicating as an integral part of the chromosome. In defense against being lost by bacteria, they have acquired in the course of evolution genes providing the bacteria harboring them with better adaptation to the occupied environments. Therefore, most temperate bacteriophages of bacterial pathogens encode virulence factors, such as *inter alia* bacterial toxins, adhesins or factors protecting the bacteria from the host immune response (Cheetham et al., 1995). Spontaneous excision of prophages from chromosomes in some cells of the population, in the process of the so-called induction, leads to initiation of their lytic development. Temperate phages multiplied during lytic development may, when infecting further bacterial cells, integrate into their DNA as prophages, in this way becoming carriers for genes associated with bacterial virulence. A variety of epidemic bacterial strains particularly difficult to fight with, has emerged through their acquisition of new prophages (Canchaya et al., 2003). For this reason, it is necessary in phage therapy to utilize only monoclonal phage preparations, containing only the therapeutic phage virions and not contaminated with other phages ( 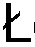 obocka et al., 2014). Simultaneously, cocktails consisting of several types of phages should be obtained only by mixing monoclonal preparations.

Another type of mobile genetic elements that may contaminate phage preparations are plasmids - extrachromosomal DNA molecules capable of autonomous replication. Pathogenic bacteria plasmids, like temperate phages, have, in the course of evolution, acquired genes associated with virulence of their bacterial hosts. Furthermore, during bacteriophage multiplication in bacterial cells, plasmids may integrate into phage DNA and as a part of this DNA be packed into virions (Masters, 1996). Replacing in this way fragments of phage DNA they give rise to defective phages, incapable of multiplication. These phages however introduce the plasmid-containing DNA into the infected cells and in this way may disseminate bacterial virulence genes. Therefore, the bacteria for multiplication of therapeutic phages should not contain plasmids. Few Staphylococcus aureus strains that do not contain prophages and plasmids are known (WO 2014/048407 and Vaudaux et al., 2002).

The aim of the invention is to provide *Staphylococcus aureus* strains for production of therapeutic bacteriophages preparations having high titer, devoid of contamination with temperate phages and plasmid DNA. Unexpectedly, the thus defined aim has been achieved due to the solution proposed by the present invention.

The object of the invention is a *Staphylococcus aureus* strain devoid of prophages and plasmids. The strain according to the invention is a *Staphylococcus aureus* 80wphwpl strain deposited in the PCM (Polish Collection of Microorganisms) as B/00072.

The preferred object of the invention is a *Staphylococcus aureus* 80wphwpl strain having a bacterial chromosome comprising DNA with a sequence SEQ ID NO: 1.

The object of the invention is also a method of producing a bacteriophage preparation, particularly a therapeutic one, characterized by the fact that a bacteriophage strain specific against *Staphylococcus aureus* is multiplied in culture of the *Staphylococcus aureus* strain devoid of prophages and plasmids according to the invention as defined above, and then the multiplied phages are isolated from the obtained lysate, wherein the obtained bacteriophage preparations are devoid of contamination with temperate phages and plasmid DNA.

The effect of the invention is to provide S. *aureus* 80wphwpl strain having genomic DNA sequences designated as SEQ ID NO: 1, characterized by the lack of sequences corresponding to genomic sequences of active prophages.

A further effect of the invention is the lack of plasmids in the 80wphwpl strain cells.

An advantageous effect of the invention is the 80wphwpl strains maintaining these parental strains features that allow for effective multiplication of *Staphylococcus aureus* bacteriophages in their cells, including in particular the P4W, Staph1N, A5W or Fi200W bacteriophages, belonging to the Twort-like type.

An additional advantageous effect of the invention is the possibility to obtain, as a result of multiplication of P4W, Staph1N, A5W or Fi200W bacteriophages in the 80wphwpl strain cells, lysates containing the aforementioned bacteriophages with a titer of at least 10⁹ pfu/ml.

The standard so far, which was considered sufficient for excluding the presence of undesired bacteriophages in therapeutic bacteriophage preparations, were the observations under an electron microscope (Merabishvili et al., 2009). In this way, around several dozens to hundreds of virions in a visual field may be viewed, verifying their morphological homogeneity. The presence of plasmids in the cells for therapeutic phage multiplication was ignored, despite reports in literature about numerous cases of plasmid transfer between cells by bacteriophages (reviewed in 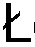 obocka et al., 2014).

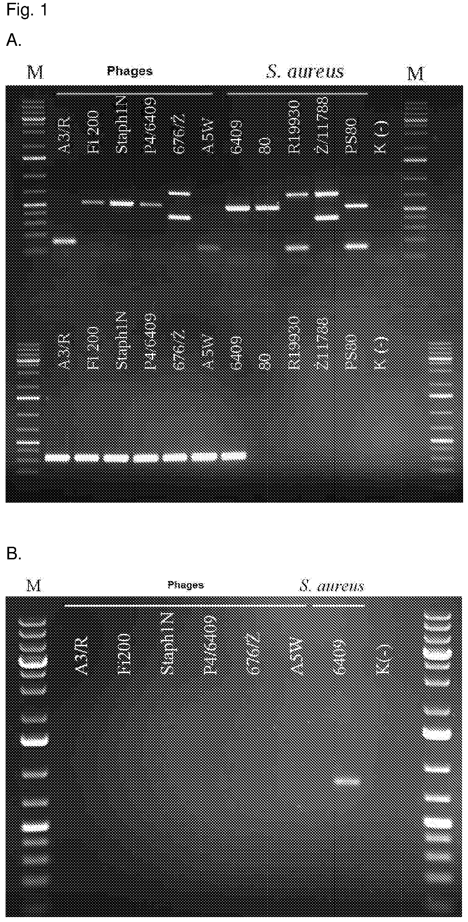

The present inventors have unexpectedly found that all of the studied preparations of obligate lytic *Staphylococcus aureus* bacteriophages of the Twort-like type, multiplied in the cells of S. *aureus* isolates enabling the production of high titer lysates of these bacteriophages, are contaminated with undesired bacteriophages, and that the source of these bacteriophages are prophages integrated into chromosomes of the strains employed to multiply phages (Fig. 1). Despite recent study results indicating the presence of prophages in genomes of almost all clinical and environmental S. *aureus* isolates, there has been no studies conducted that would allow to estimate the frequency of spontaneous induction of various S. *aureus* prophages and to evaluate the risk of contaminating the S. *aureus* culture with phages generated as a result of prophage induction (Kahankova et al., 2013). Moreover, no S. *aureus* strains devoid of prophages for multiplication of bacteriophages of the Twort-like type, which are the most valuable ones from the therapeutic point of view, have been described.

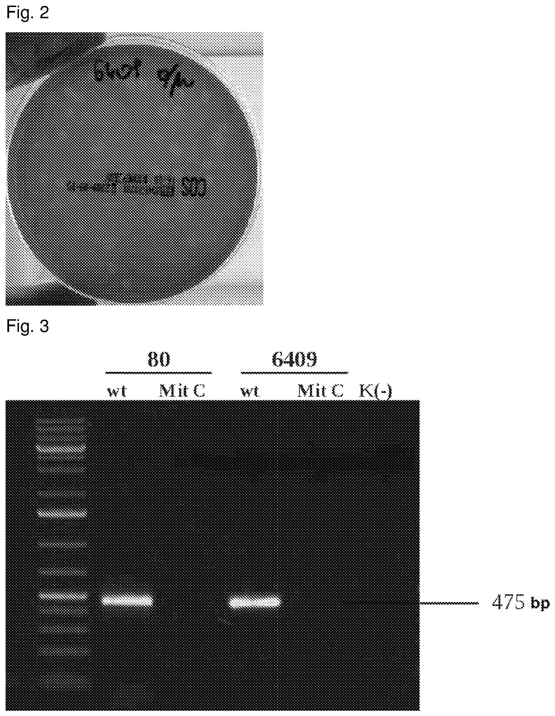

With the aim of eliminating prophages from the two *Staphylococcus aureus* strains for efficient multiplication of therapeutic phages, the inventors have determined their genomic DNA sequence. In this way they have identified the sites of prophage integration in the genomes of these strains and the genes having their continuity interrupted by integration of prophages. Having associated the phenotype with the loss of function for these genes, the inventors have identified colonies of cells that lost the prophages (Fig. 2). The loss of prophages was confirmed by sequence analysis of the cured strain genomic DNA (specified for the purposes of the present specification as SEQ ID NO: 1 and SEQ ID NO: 2). Additionally, by analyzing the total DNA sequence of the studied strains, the inventors have unexpectedly found that the cells of these strains contain plasmids that may encode *inter alia* resistance to cadmium salts. Searching for the cells of the studied strains that acquired sensitivity to cadmium in conditions that may stimulate plasmid loss, that have isolated plasmid-less derivatives of both strains and have confirmed the lack of plasmids in their cells by analyzing total DNA isolated from these cells.

In the examples described in literature it is apparent that prophages carried in bacterial cells may encode functions essential for the development of particular obligate lytic phages, rendering it impossible for these bacteriophages to multiply in the strains cured of prophages (Nirmal Kumar et al., 2012). Therefore, it was surprising to demonstrate that the *Staphylococcus aureus* 80wphwpl and 6409wphwpl strains described herein, despite them being devoid of their prophages and plasmids, may still serve as bacterial hosts for multiplication of lytic bacteriophages. Moreover, the bacteriophage titer in lysates obtained after infecting these strains with therapeutic phages does not deviate from the titer in lysates obtained after infecting with parental strain phages.

Thus, it is clear that the obtained S. *aureus* 80wphwpl and 6409wphwpl strains are the strains of choice for multiplication of bacteriophages used for prophylactic, therapeutic or disinfection purposes in fighting S. *aureus,* by guaranteeing that phage preparations, obtained by multiplying obligate lytic bacteriophages in the cells of these strains, are free from contamination with undesired biological material that would pose a risk of dissemination of bacterial virulence associated genes. This excludes the possibility of deterioration of the condition of patients or animals treated with such preparations due to an unplanned acquisition by the infecting S. *aureus* strains of additional genes associated with virulence, which cannot be excluded in the case of preparations used to date.

An embodiment of the invention is illustrated by the following figures:
Figure 1 shows detection of contamination of obligate lytic bacteriophage preparations with bacteriophages generated due to spontaneous induction of prophages in bacterial strains used for phage multiplication. (A) The top gel shows amplification products obtained in multiplex PCRs using DNA isolated from virions contained in obligate lytic phage preparations (Phages) or chromosomal DNA from S. *aureus* strains used for phage multiplication (*S. aureus*) as template, and with primers specific to DNA of temperate bacteriophages from the following serogroups: A (estimated PCR product length - 744 bp), B (estimated PCR product length - 405 bp), Fa (estimated PCR product length - 548 bp), Fb (estimated PCR product length - 147 bp). The bottom gel shows amplification products with the aforementioned templates and primers specific to temperate bacteriophages from the L serogroup (estimated PCR product length 648 bp) and to the multiplied lytic bacteriophages from the D serogroup (estimated PCR product length 331 bp). Sequence of primers used for detecting bacteriophages from individual serogroups and reaction conditions for multiplex PCR were as described previously (Pantucek et al., 2004). DNA fragments obtained in PCR reactions were separated by electrophoresis in 1% agarose gel. The lane marked with M contains separated DNA size marker fragments (GeneRulerTM 1 kb Plus DNA Ladder, Fermentas), with the following respective sizes (in base pairs): 75, 200, 300, 400, 500, 700, 1000, 1500, 2000, 3000, 4000, 5000, 7000, 10000 and 20000. In lanes identified as K(-) control mixtures were separated, not containing template DNA. (B) Amplification products that were obtained using DNA isolated from virions contained in obligate lytic phage preparations (Phages) or chromosomal DNA of the S. *aureus* 6409 strain (used in part A) as template and with primers specific to the S. *aureus* chromosomal coagulase gene.
Figure 2 shows selection of the 6409 strain derivatives that acquired the ability to hemolysis after mitomycin C treatment. Cells treated with mitomycin C were plated on Columbia agar medium with sheep blood. Hemolysis zones are visible as clearings around the colonies.
Figure 3 shows the negative result of PCR utilizing as template the DNA of selected derivatives of the 80 and 6409 strains that have recovered the ability to hemolyze sheep blood erythrocytes, after mitomycin C treatment (lanes marked MitC), and with primers specific for the Fa serogroup prophages, present in genomes of the parental strains (lanes marked wt). The employed amplicon separation conditions, primers specific to the Fa serogroup prophages and the type of DNA size marker are the same as given in the description of Fig. 1.

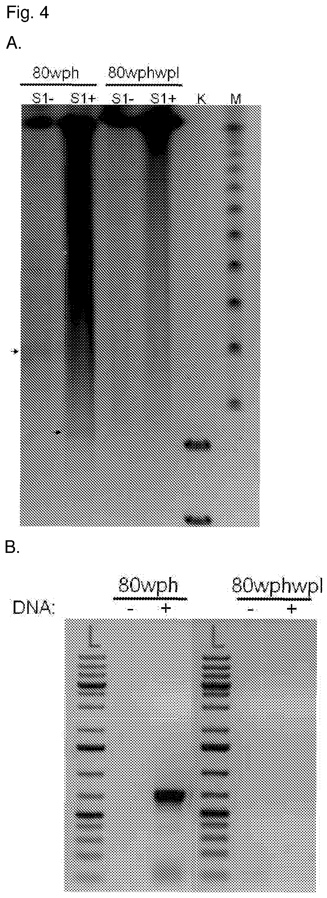

Figure 4 shows the results confirming the lack of plasmid in the 80wph strain derivative, designated as 80wphwpl. (A) The separation in agarose gel by the PFGE method of DNA from the 80wph strain and the 80wphwpl derivative thereof lacking the plasmid.

Arrows indicate the undigested and S1 nuclease-digested plasmid DNA present in the 80wph strain cells. The employed conditions for digestion of DNA preparations with S1 nuclease and for PFGE separation were analogous to the ones described previously (Barton et al., 1995).

In gel lanes marked S1- and S1+ undigested and S1 nuclease-digested DNA was separated, in lanes marked K+ and M purified control plasmid DNA isolated from another strain and DNA size marker fragments (Lambda Ladder PFGE Marker, New England Biolabs) were separated, respectively. (B) The separation of amplification products obtained using the 80wph or 80wphwpl strain total DNA as template and the following primers: 5'-TTCAGTAATAAACATTTGTGCGA and 5'-CATGTTTTTACGGCGCATAT, specific to plasmids present in the 80wph and 6409wph strain cells. Lanes marked - did not contain template DNA. In lanes marked L DNA size marker fragments (GeneRulerTM 1 kb Plus DNA Ladder, Fermentas) were separated. Amplification was performed using a temperature gradient for primer hybridization with the template in the range of 49-59°C.

For a better understanding of the essence of the invention it is illustrated by the following examples that demonstrate the method of detection of phage preparations contamination with undesired temperate bacteriophages, the method of prophages and plasmids elimination from the strains for multiplication of therapeutic phages and the efficient multiplication of therapeutic bacteriophages in the acquired strains devoid of plasmids and prophages. By disclosing genomic DNA sequences of the obtained strains and demonstrating the lack of plasmids in the cells thereof, they also prove that these strains cannot be a source of contamination with temperate bacteriophages and plasmids for lytic bacteriophages multiplied in their cells. In all examples, unless stated otherwise, standard molecular biology methods were employed, described by Sambrook et al. (1989).

Example 1. The presence of undesired contamination with bacteriophages in obligate lytic bacteriophage preparations multiplied in *Staphylococcus aureus* cells.

It was tested whether the obligate lytic phage preparations employed in phage therapy and multiplied in the cells of *Staphylococcus aureus* strains normally used for multiplication of these phages ( 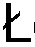 obocka et al., 2012) are contaminated with other phages. Lysates obtained after infecting cells of the following strains: 6409, 80, R19930, Z11788 and PS80 with the following phages: A3R (strain R19930), Fi200W or P4W (strain 6409), Staph1N (strain 80), 676Z (strain Z11788) and A5W (strain PS80) were purified from bacterial DNA and RNA by digesting with DNase and RNase, and subsequently the virions and their DNA were isolated therefrom according to the previously described procedure ( 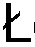 obocka et al., 2004). Concurrently, chromosomal DNA was isolated from non-infected cells of the strains used for phage multiplication. Purified virion DNA and purified chromosomal DNA of the strains for phage multiplication were used as templates for amplification of fragments representative for S. *aureus* temperate bacteriophages, representing various serotypes (Fig. 1A), using known primer sequences (Pantucek et al., 2004). Unexpectedly, it was found that all of the studied obligate lytic bacteriophage preparations were contaminated with DNA representing the genomes of temperate phages, whose prophages were integrated into chromosomes of the strains for phage multiplication. Since the phage preparations, before virion DNA isolation, were purified from free DNA from bacterial chromosome debris in the lysates, so that in control PCRs with primers for the S. *aureus* chromosomal coagulase gene no PCR product was obtained (Fig. 1B), the only source of the temperate bacteriophage DNA detected in these preparations could have been the virions of these bacteriophages, generated as a result of spontaneous induction of prophages integrated into chromosomes of the strains for multiplication of lytic phages.

Example 2. Identification of prophage integration regions in the S. *aureus* 80 and 6409 strains.

Selected for prophage curing were the 80 and 6409 strains. Obligate lytic phage lysates multiplied in these strain cells were contaminated with phages representing the Fa serological type (Fig. 1). In order to identify prophage integration regions in the genomes of the 80 and 6409 strains, chromosomal DNA was isolated from cells of these strains and it was used to prepare libraries of fragments that were subsequently sequenced (using Roche 454 Genome Sequencer GS FLX), and sequences thereof were assembled into larger fragments. Bioinformatic analysis of the obtained fragments regarding the regions homologous to the prophage DNA regions has shown the presence of prophages representing the Fa serological group in the *hlb* gene, both in the 80 and the 6409 strain. The identified prophages were interrupting the continuity of the *hlb* gene wherein they had integrated. The prophage integration regions were verified by phenotypic analysis of the studied strains. The 80 and 6409 strains were found to be incapable of hemolyzing sheep blood erythrocytes on the Columbia agar medium, which confirmed the loss of function of the *hlb* gene in these strains' genomes. Example 3. Deprivation of the S. *aureus* 80 and 6409 strains of active prophages contained in their chromosomes.

The prophages contained in the chromosomes of the 80 and 6409 strains were induced with mitomycin C according to the standard procedure. Following the induction, the cells were plated on a solid Columbia agar medium with sheep blood (Fig. 2). The obtained derivatives of strains 80 and 6409 (designated as 80wph and 6409wph), having a marked hemolytic zone around them, were purified several times by streaking on the same medium. Unexpectedly, only a few of them did retain the phenotype indicating a permanent recovery of the *hlb* gene function. One colony for each strain was used to isolate genomic DNA. Multiplex PCRs with the isolated DNA for each strain and with primers specific for the temperate bacteriophages of the Fa serogroup demonstrated no amplification products for prophage DNA fragments that in the case of parental strains were contaminating the obligate lytic phage preparations prepared in cells thereof (Fig. 3). In order to confirm the elimination of prophages from the genomes of the aforementioned strains, their total DNA was sequenced according to the previously described procedure for phage genomes ( 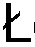 obocka et al., 2012). The obtained genomic sequences for the strains 80wph and 6409wph (designated for the purposes of this invention as SEQ ID NO: 1 and SEQ ID NO: 2 respectively) were analyzed for the presence of DNA fragments therein representing the active prophages. The analysis confirmed the lack of such regions.

Example 4. Removal of plasmids from cells of the 80wph and 6409wph strains.

Analysis of total DNA isolated from the 80wph and 6409wph strain cells allowed to distinguish, in both these strains, aside from chromosomal DNA, additional, homologous relative to one another, circular DNA molecules with a size of 20 000 bp, representing plasmids that may encode resistance to cadmium salts. The resistance of the 80wph and 6409wph strains to cadmium salts was confirmed in phenotypic tests and was used for selecting the cells that had lost the plasmid. The frequency of a spontaneous loss of plasmid was enhanced by culturing the cells at an elevated temperature. Overnight cultures of the 80wph and 6409wph strains were diluted four-fold in LB medium, incubated for 1,5 h with shaking at 37°C, whereupon the culture was diluted again about 100-fold in fresh LB medium and was incubated for 5.5 h with shaking at 43°C. Culture bacteria were diluted 10⁻⁴ and were plated on solid LB medium, so as to obtain individual colonies. The plates were incubated overnight at 37°C. The grown colonies were replicated on LB medium with cadmium acetate (67 µg/ml) and without cadmium acetate and were incubated overnight at 37°C. The colonies unable to grow on medium with cadmium acetate were purified several times by streaking. The lack of plasmid in the obtained isolates, designated as 80wphwpl and 6409wphwpl respectively, was verified by isolating total DNA therefrom, by separating the obtained DNA in agarose gel by the pulsed field electrophoresis (PFGE) method or by verifying the presence of plasmid-characteristic sequences in the obtained DNA, by amplification with primers specific to plasmid DNA (Fig. 4).

The obtained *Staphylococcus aureus* strains, devoid of prophages and plasmids, were deposited in the Polish Collection of Microorganisms (PCM), Wroc 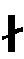 aw, Poland, according to the Budapest Treaty.

| *Staphylococcus aureus* strain designation | Polish Collection of Microorganisms (PCM) deposit accession number |
|---|---|
| 80wphwpl | B/00072 |
| 6409wphwpl | B/00073 |

Example 5. Verification of usefulness of the 80wphwpl and 6409wphwpl strains for efficient multiplication of obligate lytic phages.

In order to verify whether the 80wphwpl and 6409wphwpl strains, devoid of prophages and plasmids, may serve as host strains for multiplication of obligate lytic staphylococcal bacteriophages, and whether the bacteriophages multiply in the cells of these strains with efficiency sufficient to use them for the purposes of prevention, therapy and disinfection, the cells of the obtained strains were infected with the same bacteriophages that efficiently multiplied in the parental strain cells, carrying plasmids and prophages. In order to prepare lysates an overnight culture of each strain was diluted at a ratio of 1:100 in 50 ml of fresh LB medium and was incubated at temperature of 37°C with shaking until reaching optical density OD₆₀₀ₙₘ=∼0.3. Next, MgSO₄ and CaCl₂ ions were added to the culture (to final concentration of 10 mM) along with phage-containing lysate, so that the multiplicity of infection (M.O.I.) was 0.1. The mixtures were allowed to stand for 5 minutes at room temperature to adsorb the phages to the surface of bacterial cells and then were incubated at temperature of 37°C with shaking until observing clear indications of lysis. The lysate was centrifuged (20 min., 4°C, 9000 g) and the obtained supernatant was filtered through 0.22 PVDF filters (from MILLEX® GS). For phage titration in the purified lysate, 0.1 ml of 25 mM CaCl₂ and MgSO₄ solution and 0.1 ml of diluted phage lysate (10⁻², 10⁻⁴, 10⁻⁶, 10⁻⁸) were added to 0.1 ml of an overnight bacterial culture grown on LB medium. The mixture was allowed to stand for 5 minutes at room temperature and then 1 ml of LB medium and 4 ml of LCA medium (LB, 5 mM CaCl₂, 0.7% agar) liquidized and cooled to 55°C were added and it was poured onto the surface of LB medium plates. After overnight incubation at temperature of 37°C, plaques in the bacterial cell layer were counted and on that basis the number of infecting phage particles per 1 ml of lysate was determined. In all cases the titer of the obtained phages in the lysates of strains devoid of plasmids and prophages was comparable to the titer thereof in the lysates of parental strains (Table 1). At the same time, they were consistent with titer range for therapeutically applied staphylococcal bacteriophages in lysates (Merabishvili et al., 2009), which indicates unambiguously the usefulness of the strains obtained according to the present invention for production of phage preparations for therapeutic purposes.

**Table 1. Phage titer in lysates of infected cells of strains devoid of prophages and plasmids and of parental strains.**

| **Bakteriophage** | **Strain for multiplication** | **Phage titer in lysate** |
|---|---|---|
| A5W | 80wphwpl | 5.5 x 10⁹ |
| | 80 | 3.4 x 10⁹ |
| Staph1N | 80wphwpl | 3.0 x 10⁹ |
| | 80 | 1.5 x 10¹⁰ |
| Fi200W | 6409wphwpl | 1.8 x 10⁹ |
| | 6409 | 1.1-5.0 x 10⁹ |
| P4W | 6409wphwpl | 3.3 X 10⁹ |
| | 6409 | 1.5 x 10¹⁰ |

### Literature:

Balogh, B.; Jones, J. B.; Iriarte, F. B.; Momol, M. T. Phage therapy for plant disease control. Curr. Pharm. Biotechnol., 2010, 11: 48-57.
Barton BM, Harding GP, Zuccarelli AJ. 1995. A general method for detecting and sizing large plasmids. Anal. Biochem. 226: 235-240.
Canchaya C, Proux C, Fournous G, Bruttin A, Brüssow H. 2003. Prophage genomics. Microbiol. Mol. Biol. Rev. 67: 238-276,
Cheetham BF, Katz ME. 1995. A role for bacteriophages in the evolution and transfer of bacterial virulence determinants. Mol Microbiol. 18: 201-208.
D'Herelle. Preparation of Therapeutic Bacteriophages, Appendix 1 from: Le Phenomene de la Guerison dans les maladies infectieuses. 1938. Masson et Cie, 1938, Paris-OCLC 5784382. (translation from Bacteriophages 2011; 1, 55-65).
Gill, J.J., and Hyman, P. 2010. Phage choice, isolation, and preparation for phage therapy. Curr Pharm Biotechnol. 11: 2-14.
Golkar Z, Bagasra O, Pace DG. 2014. Bacteriophage therapy: a potential solution for the antibiotic resistance crisis. J Infect Dev Ctries. 2014 Feb 13;8(2):129-36.
Guttman, B., Raya, P., and Kutter, E. 2005. Basic phage biology. In Bacteriophages: biology and application, E.B., Kutter, and A., Sulakvelidze, eds. (Boca Raton (FL): CRC Press), pp. 29-66.
Kahankova, J., Pantucek, R., Goerke, C., Ruzickova, V., Holochova, P., Doskar, J. 2010. Multilocus PCR typing strategy for differentiation of Staphylococcus aureus siphoviruses reflecting their modular genome structure. Environ. Microbiol. 12: 2527-2538.
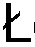 obocka, M.B., Rose, D.J., Plunkett, G. 3rd, Rusin, M., Samojedny, A., Lehnherr, H., Yarmolinsky, M.B., and Blattner, F.R. 2004. Genome of bacteriophage P1. J. Bacteriol 186, 7032-7068.
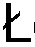 obocka, M., Hejnowicz, M.S., D 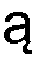 browski, K., Gozdek, A., Kosakowski, J., Witkowska, M., Ulatowska, M.I., Weber-D 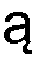 browska, B., Kwiatek, M., Parasion, S., Gawor, J., Kosowska, H., G 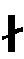 owacka, A. 2012. Genomics of staphylococcal Twort-like phages-potential therapeutics of the post-antibiotic era. Adv Virus Res. 83, 143-216.
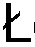 obocka M., Hejnowicz M. S., G 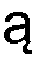 ga 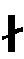 a U., Weber-D 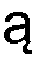 browska B., W 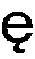 grzyn G., Dadlez M. 2014. The first step to bacteriophage therapy - how to choose the correct phage. In.: Phage Therapy. Current Research and Applications. Borysowski J., Mi 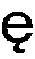 dzybrodzki R., Górski, A. (ed.). Caister Academic Press. pp. 23-69.
Masters, M. 2004. Transduction: host DNA transfer by bacteriophages. In The Desk Encyclopedia of Microbiology, M. Schaechter, eds. (London, UK: Elsevier Academic Press), pp. 1000-1012.
Merabishvili, M., Pirnay, J.P., Verbeken, G., Chanishvili, N., Tediashvili, M., Lashkhi, N., Glonti, T., Krylov, V., Mast, J., Van Parys, L., Lavigne, R., Volckaert, G., Mattheus, W., Verween, G., De Corte, P., Rose, T., Jennes, S,, Zizi, M., De Vos, D., and Vaneechoutte, M. 2009. Quality-controlled small-scale production of a well-defined bacteriophage cocktail for use in human clinical trials. PLoS One. 4, e4944.
Nirmal Kumar GP, Sundarrajan S, Paul VD, Nandini S, Saravanan RS, Hariharan S, Sriram B, Padmanabhan S. 2012. Use of prophage free host for achieving homogenous population of bacteriophages: new findings. Virus Res. 169: 182-187.
Pantůcek R, Doskar J, Růzicková V, Kaspárek P, Orácová E, Kvardová V, Rosypal S. 2004. Identification of bacteriophage types and their carriage in Staphylococcus aureus. Arch Virol. 149: 1689-1703.
Vaudaux P et al. 2002. Increased expression of clumping factor and fibronectin-binding proteins by hemB mutants of Staphylococcus aureus expressing small colony variant phenotypes. Infect Immun. 70: 5428-37.

## Claims

1. A *Staphylococcus aureus* strain devoid of prophages and plasmids, **characterized in that** it is a *Staphylococcus aureus* 80wphwpl strain deposited in the PCM as B/00072.

2. A *Staphylococcus aureus* strain according to claim 1 **characterized in that** it has a bacterial chromosome comprising DNA with a sequence SEQ ID NO: 1.

3. A method of producing a bacteriophage preparation, particularly a therapeutic one, **characterized in that** a bacteriophage strain specific against *Staphylococcus aureus* is multiplied in culture of the *Staphylococcus aureus* strain devoid of prophages and plasmids, according to claims 1-2, and then the multiplied phages are isolated from the obtained lysate, wherein the obtained bacteriophage preparations are devoid of contamination with temperate phages and plasmid DNA.

## Patentansprüche

1. *Staphylococcus aureus* Stamm frei von Prophagen und Plasmiden, **dadurch gekennzeichnet, dass** er der Stamm *Staphylococcus aureus* 80wphwpl wie in der PCM als B/00072 hinterlegt ist.

2. *Staphylococcus aureus* Stamm nach Anspruch 1, **dadurch gekennzeichnet, dass** er ein bakterielles Chromosom umfassend eine DNA mit einer Sequenz SEQ ID NO: 1 aufweist.

3. Verfahren zur Herstellung einer Bakteriophagenpräparation, insbesondere einer therapeutischen Bakteriophagenpräparation, **dadurch gekennzeichnet, dass** ein gegen *Staphylococcus aureus* spezifischer Bakteriophagenstamm in einer Kultur des *Enterococcus faecalis* Stammes frei von Prophagen und Plasmiden nach den Ansprüchen 1 - 2 vervielfältigt wird, und dann die vervielfältigten Phagen von dem erhaltenen Lysat isoliert werden, wobei die erhaltenen Bakteriophagenpräparationen frei von einer Kontamination mit temperenten Phagen und Plasmid-DNA sind.

## Revendications

1. Souche de *Staphylococcus aureus* dépourvue de prophages et de plasmides, **caractérisée en ce qu'**il s'agit d'une souche de *Staphylococcus aureus* 80wphwpl déposée auprès de la CPM en tant que B/00072.

2. Souche de *Staphylococcus aureus selon* la revendication 1, **caractérisée en ce qu'**elle a un chromosome bactérien comprenant un ADN avec une séquence SEQ ID N° : 1.

3. Procédé de production d'une préparation de bactériophages, en particulier une préparation thérapeutique, **caractérisé en ce qu'**une souche bactériophage spécifique dirigée contre *Staphylococcus aureus* est multipliée lors de la culture de la souche de *Staphylococcus aureus* dépourvue de prophages et de plasmides, selon les revendications 1-2, puis les phages multipliés sont isolés du lysat obtenu, dans lequel les préparations de bactériophages obtenues sont dépourvues de contamination avec des phages tempérés et de l'ADN plasmidique.
